# EUROPEAN PATENT APPLICATION

(11) **EP 4 129 472 A1**
(43) Date of publication of application: **08.02.2023**
(21) Application number: 21382736.3
(22) Date of filing: 05.08.2021
(51) Int. Cl.: B01J 23/83, B01J 35/10, B01J 37/18, B01J 37/08, B01J 37/04, C10L 3/08, C07C 1/12, B01J 35/02, B01J 37/02, B01J 21/04

(54) **A NICKEL MICRO-CATALYST, A PROCESS FOR PREPARING THEREOF AND USE THEREOF**

(71) Applicant: Fundació Institut de Recerca de l'Energía de Catalunya, 08930 Sant Adrià del Besòs (ES)
(72) Inventor: GUILERA SALA, Jordi, Vilafranca del Penedès (ES); ANDREU ARBELLA, Teresa, Sant Boi de Llobregat (ES); ALARCÓN AVELLÁN, Andreína Alexandra, Barcelona (ES); BADIA CÓRCOLES, Jordi Hug, Barcelona (ES)
(74) Representative: Ponti & Partners, S.L.P

(57) **Abstract**

The present invention relates to a micro-catalyst consisting of nickel (Ni) and a compound selected from cerium oxide (CeO₂), lanthanum oxide (La₂O₃) or a combination thereof, using aluminium oxide (γ-Al₂O₃) as a support. The present invention also relates to a process for preparing said micro-catalyst by melting Infiltration under vacuum and its use for producing synthetic methane by the carbon dioxide and hydrogen conversion into methane and water.

## Description

### Field of the invention

The present invention relates to the field of synthetic production of methane. In particular, the present invention relates to a micro-catalyst useful for the conversion of hydrogen and carbon dioxide to methane, and to a process for preparing thereof.

### Background

The combination of carbon dioxide (CO₂) and hydrogen (H₂) by the presence of an active catalyst generates synthetic methane (CH₄) and water (H₂O); and releases heat (-165 kJ/mol). The reaction, also referred herein as "CO₂ methanation" is described as follows:

CO₂ + 4 H₂ ↔ CH₄ + 2 H₂O ΔᵣH=-165 kJ/mol

Industrial interest in CO₂ methanation reaction lies in the fact that the product, synthetic methane, has a similar composition as natural gas, and thus, synthetic methane can be transported and distributed in the existing gas infrastructures; and further consumed in the same applications as natural gas. The main difference between natural gas and synthetic methane is that natural gas is a fossil fuel (non-renewable) extracted from deep underground rock formations or associated with other hydrocarbon reservoirs, whereas synthetic methane is artificially synthesized. In the case that the raw materials, CO₂ (biogenic) and H₂ (green) are renewable, synthetic methane is also renewable.

The CO₂ methanation reaction is exothermic and reversible. Thermodynamic equilibrium conversion is favoured at low temperature, whereas the chemical reaction is kinetically limited at low temperatures. Thus, a compromise between favourable kinetics and equilibrium, as well as catalyst thermal deactivation is considered. High pressure favours both the kinetics and the thermodynamics, while it increases the energy consumption. Commonly, the reaction is carried out at moderate temperatures (T=200-600°C) and lowto-moderate pressures (P=1-25 bar·g).

A challenge in the design of CO₂ methanation reactor is to properly control the reaction temperature inside the reactive channel. Channel geometry at macroscopic scale (> 1mm) limits operation at high gas flow rates because of undesired radial temperature profiles. This aspect can be overcome by reducing the lateral dimensions to microscopic scale (<1 mm), leading to the intensification of the process. Short distances combined with high surface to volume ratio are the key to excellent temperature control. Micro-reactor technology, defined as a reactor with at least 1 dimension below 1 mm, seems to be the most appropriate for CO₂ methanation in terms of process intensification.

A CO₂ methanation catalyst placed inside the micro-reactor should be able to accomplish with the dimension restrictions (<1 mm), also named herein as micro-catalyst. Besides its suitable incorporation to more efficient devices, micro-catalysts benefits from restricted heat and mass transfer resistances when compared to conventional macro-catalyst, and thus, the reaction rates are increased. A highly active formulation for CO₂ methanation catalyst is of special interest for micro-reactor applications because the rest of resistances are already minimized and the intrinsic chemical reaction rate gains importance. The sum of the benefits from micro-reactor and micro-catalyst technology increases the final yield to synthetic methane. A higher yield can be expressed as higher conversion, higher selectivity, lower contact time or/and lower reactor volumes.

The present inventors have found that the micro-catalyst as disclosed in the present invention is surprisingly suitable and very active for the conversion of hydrogen and carbon dioxide to synthetic methane, preferably when the micro-catalyst is prepared by the vacuum melting infiltration process as disclosed in the present invention.

### Summary of the invention

In a first aspect, the present invention relates to a micro-catalyst as defined in the set of claims.

In a second aspect, the present invention relates to a process for preparing a micro-catalyst according to the first aspect of the invention.

In a third aspect, the present invention relates to the use of a micro-catalyst according to the first aspect of the invention.

### Brief description of the drawings

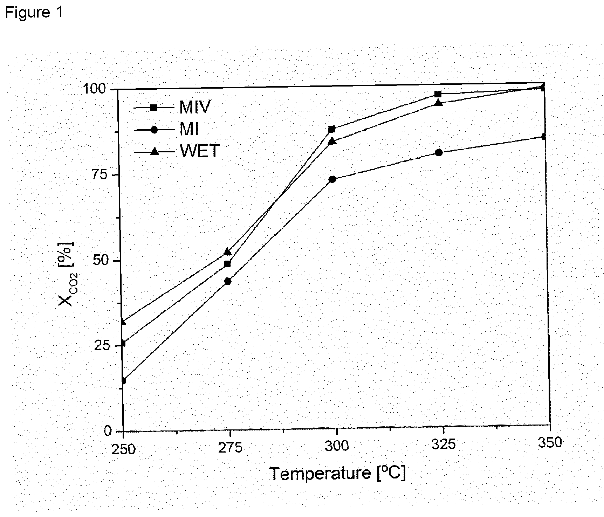

Figure 1. This figure represents CO₂ conversions in the temperature range of 250 to 350°C obtained using an isothermal fixed-bed reactor and a micro-catalyst prepared with 20 wt.% of nickel and 15 wt.% of cerium oxide. The catalytic activity test of the micro-catalysts was carried out under isothermal conditions. With this figure the comparison between the Melting Infiltration under Vacuum method (MIV) of the present invention and alternative methods such as conventional Melting Infiltration (MI) and Wet Impregnation (WET) using the same formulation is further shown.

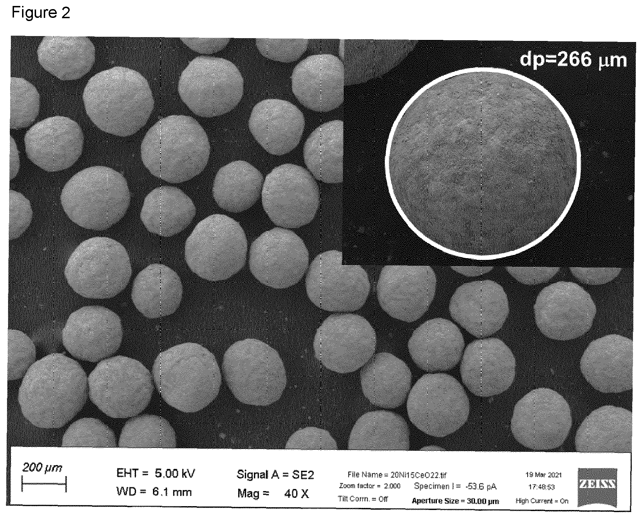

Figure 2. Scanning electron microscopy (SEM) image of the micro-catalyst developed on the base of the microspheres of alumina that were impregnated with 20 wt.% of nickel and 15 wt.% of cerium oxide, showing its spherical geometric shape and particle diameter of 266 µm.

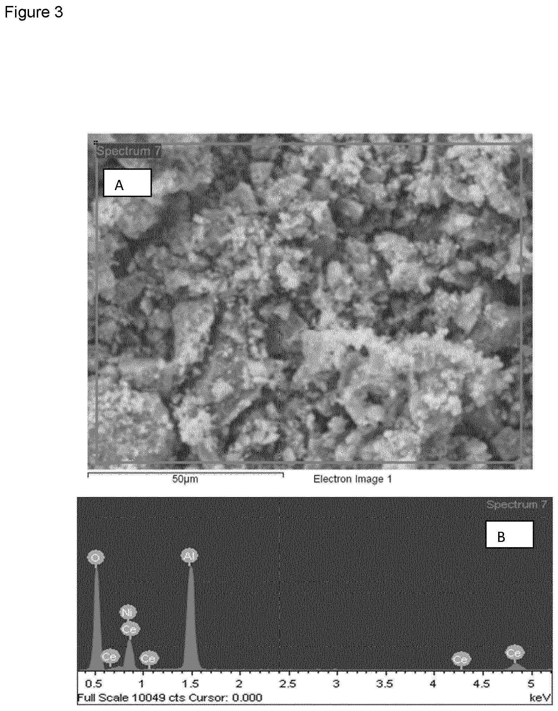

Figure 3. SEM image of a sample surface area (A) and energy dispersive X-rays (EDX) spectrum (B) of the micro-catalyst developed on the base of the microspheres of alumina that were impregnated with 20 wt.% of nickel and 15 wt.% of cerium oxide.

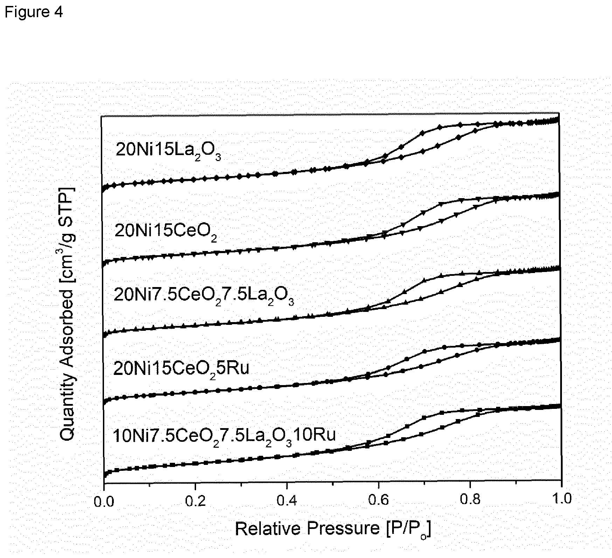

Figure 4. N₂-physisorption (adsorption/desorption) isotherms of the series of micro-catalysts developed on the base of the microspheres of alumina that were impregnated with nickel, ruthenium, cerium oxide and lanthanum oxide, corresponding the isotherm profiles (from top to bottom) to 20 wt.% Ni-15 wt.% La₂O₃; 20 wt. %Ni-15 wt.% CeO₂; 20 wt.% Ni-7.5 wt.% CeO₂-7.5 wt.% La₂O₃; 20 wt.% Ni-15 wt.% CeO₂-5wt.% Ru; and, 10wt.% Ni-7.5 wt.% CeO₂-7.5 wt.% La₂O₃-10 wt.% Ru. Formulations with Ru are used for comparative purposes.

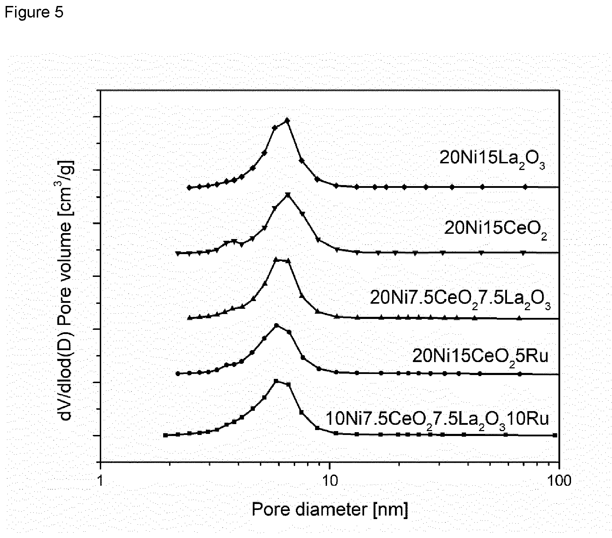

Figure 5. Distribution of the pore diameter, determined with the BJH (Barrett-Joyner-Halenda) desorption cumulative plots of the series of micro-catalysts developed on the base of the microspheres of alumina that were impregnated with nickel, ruthenium, cerium oxide and lanthanum oxide, corresponding the profiles (from top to bottom) to 20 wt.% Ni-15 wt.% La₂O₃; 20 wt. %Ni-15 wt.% CeO₂; 20 wt.% Ni-7.5 wt.% CeO₂-7.5 wt.% La₂O₃; 20 wt.% Ni-15 wt.% CeO₂-5 wt.% Ru; and, 10 wt.% Ni-7.5 wt.% CeO₂-7.5 wt.% La₂O₃-10 wt.% Ru. Formulations with Ru are used for comparative purposes.

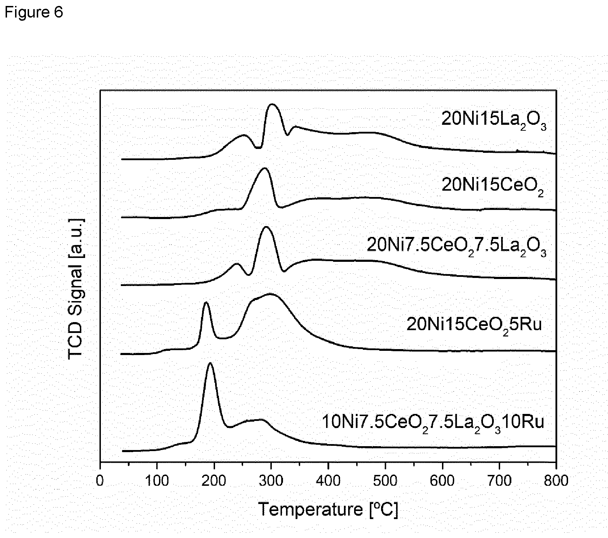

Figure 6. H₂-TPR (Temperature Programmed Reduction) profile of the series of micro-catalysts developed on the base of the microspheres of alumina that were impregnated with nickel, ruthenium, cerium oxide and lanthanum oxide, corresponding the profiles (from top to bottom) to 20 wt.% Ni-15 wt.% La₂O₃; 20 wt. %Ni-15 wt.% CeO₂; 20 wt.% Ni-7.5 wt.% CeO₂-7.5 wt.% La₂O₃; 20 wt.% Ni-15 wt.% CeO₂- 5 wt.% Ru; and, 10 wt.% Ni-7.5 wt.% CeO₂-7.5 wt.% La₂O₃-10 wt.% Ru. Formulations with Ru are used for comparative purposes.

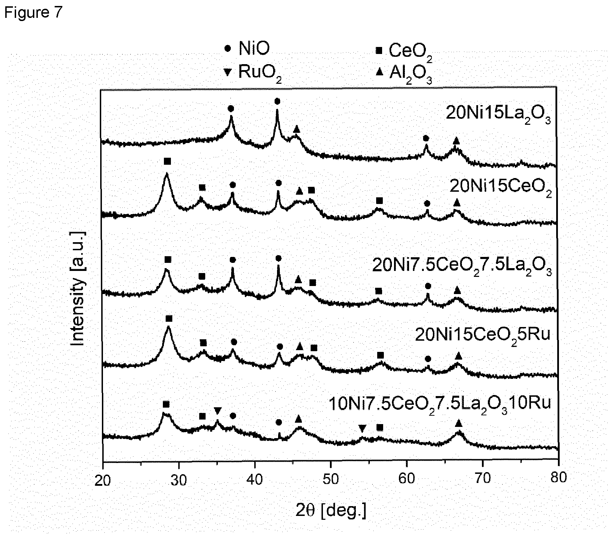

Figure 7. XRD (X-ray diffraction) pattern of the series of micro-catalysts developed on the base of the microspheres of alumina that were impregnated with nickel, ruthenium, cerium oxide and lanthanum oxide, corresponding the profiles (from top to bottom) to 20 wt.% Ni-15 wt.% La₂O₃; 20 wt. %Ni-15 wt.% CeO₂; 20 wt.% Ni-7.5 wt.% CeO₂-7.5 wt.% La₂O₃; 20 wt.% Ni-15 wt.% CeO₂- 5 wt.% Ru; and, 10 wt.% Ni-7.5 wt.% CeO₂-7.5 wt.% La₂O₃-10 wt.% Ru. Formulations with Ru are used for comparative purposes.

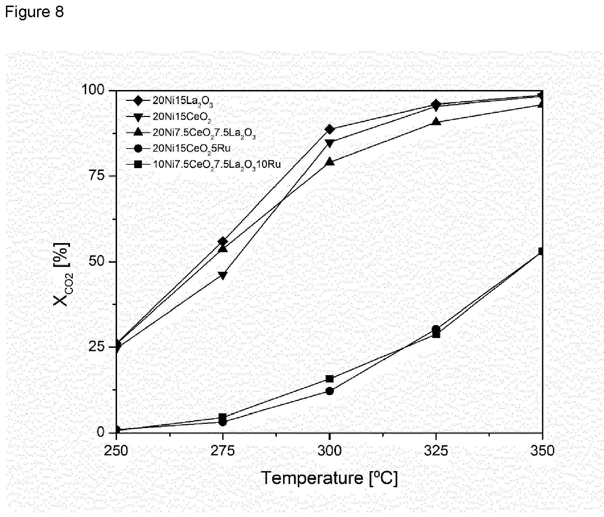

Figure 8. Catalytic activity in terms of CO₂ conversion of the series of micro-catalysts developed on the base of the microspheres of alumina that were impregnated with nickel, ruthenium, cerium oxide and lanthanum oxide, corresponding the diamond (◆) symbol to 20 wt.% Ni-15 wt.% La₂O₃; the down triangle (▼) symbol to 20 wt. %Ni-15 wt.% CeO₂; the up triangle (▲) symbol to 20 wt.% Ni-7.5 wt.% CeO₂-7.5 wt.% La₂O₃; the circle (●) symbol to 20 wt.% Ni-15 wt.% CeO₂- 5 wt.% Ru; and, the square (■) symbol to 10 wt.% Ni-7.5 wt.% CeO₂-7.5 wt.% La₂O₃-10 wt.% Ru). Formulations with Ru are used for comparative purposes.

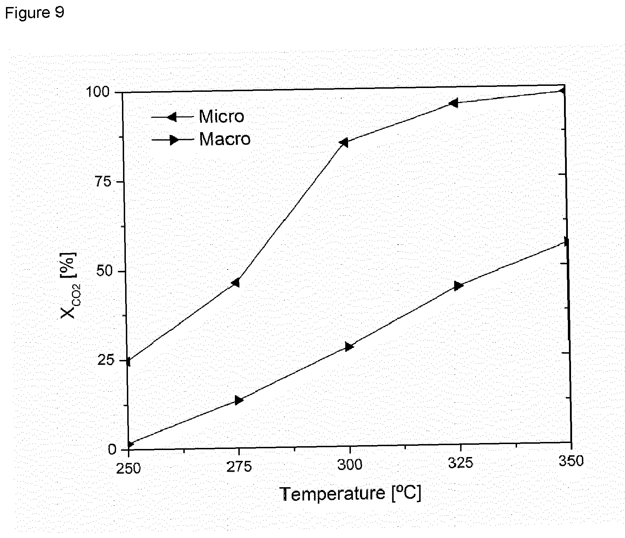

Figure 9. Comparison of the catalytic activity in terms of CO₂ conversion between two catalysts (both with the formulation 20 wt.% Ni-15 wt.% CeO₂) developed on the base of the spheres of alumina with different diameter particles, corresponding the left triangle (◄) symbol to the micro (100-300 µm) size catalyst, and the right triangle (►) symbol to the macro (2 mm) size catalyst.

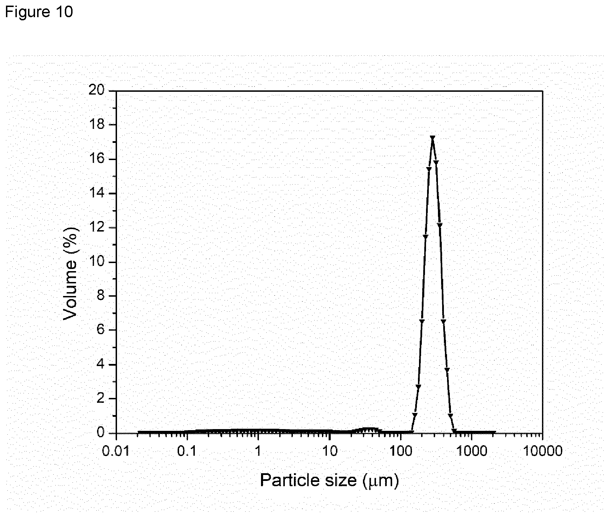

Figure 10. Particle size distribution of the micro-catalyst developed on the base of the microspheres of alumina that were impregnated with 20 wt.% of nickel and 15 wt.% of cerium oxide.

### Detailed description of the invention

In a first aspect, the present invention relates to a micro-catalyst comprising or consisting of nickel (Ni) and a compound selected from cerium oxide (CeO₂), lanthanum oxide (La₂O₃) or a combination thereof, using aluminium oxide (γ-Al₂O₃) as a support, having said micro-catalyst an average particle size between 100 and 900 µm, preferably between 100 and 800 µm, more preferably between 100 and 700 µm, even more preferably between 100 and 600 µm, further even more preferably between 100 and 500 µm, still even more preferably between 100 and 400 µm and most preferably between 100 and 300 µm,
wherein the Ni and said compound selected from CeO₂, La₂O₃ or a combination thereof, have a metal precursor salt with a meting point below 150°C and lower than its decomposition temperature, being the metal Ni or Ce o La.

In a preferred embodiment, the Ni is present at 10-30 wt % and the combination of CeO₂ and La₂O₃ (i.e CeO₂ alone if La₂O₃ is not present, La₂O₃ alone if CeO₂ is not present, or CeO₂ and La₂O₃ in combination when both are present) is present with a total amount corresponding to 5-25 wt % with respect to the total composition, being the rest aluminium oxide (γ-Al₂O₃).

In a further preferred embodiment, the Ni is present at 15-25 wt % and the combination of CeO₂ and La₂O₃ (i.e CeO₂ alone if La₂O₃ is not present, La₂O₃ alone if CeO₂ is not present, or CeO₂ and La₂O₃ in combination when both are present) is present with a total amount corresponding to 15-20 wt % with respect to the total composition, being the rest aluminium oxide (γ-Al₂O₃).

In another embodiment, the micro-catalyst has a surface area of 100-200 m²/g.

In another embodiment, the micro-catalyst has a pore volume of 0.2-0.4 cm³/g.

In another embodiment, the micro-catalyst has a pore size of 2-50 nm, preferably of 5-9 nm.

The metal precursor salt as used herein, being the metal Ni, Ce or La, is defined as a salt whose melting point is lower than its decomposition temperature, preferably said melting point is lower than 150 °C, which after chemical or physical processes, would correspondingly provide Ni, CeO₂, La₂O₃ or a combination thereof, i.e. a Ni precursor salt would provide Ni, a Ce precursor salt would provide CeO₂ and a La precursor salt would provide La₂O₃. Preferably, the anion of said salt is selected from nitrate hydrate, chloride, acetate, sulphate, ammonium sulfate, iodate, oxalate or perchlorate, more preferably nitrate hydrate.

Meting point is defined herein as the temperature at which a substance or compound changes state from solid to liquid (at ambient pressure).

The decomposition temperature is defined herein as the temperature at which a substance or compound chemically decomposes.

In a second aspect, the present invention relates to a process for preparing a micro-catalyst according to first aspect of the invention including all the embodiments alone or in combination, comprising the following steps:
a) preparing the micro-catalyst support by drying γ-Al₂O₃ at 90-110 °C;
b) mixing the metal precursor salt as previously defined, of nickel and a compound selected from cerium oxide (CeO₂), lanthanum oxide (La₂O₃) or a combination thereof, with the support of step a);
c) heating the mixture of step b) at a temperature of 80-150 °C, preferably for 0.5-3 hours;
d) cooling down the mixture of step c) at 70-90 °C;
e) drying the mixture of step d) at 70-90 °C under vacuum (preferably at P < 0.8 bar) for 3-15 hours;
f) heating the mixture obtained in step e) at 400-500 °C under air;
g) activating the mixture as obtained in step f) by flowing a stream comprising hydrogen at 300-500°C.

This process according to the present invention is named as "Melting Infiltration under Vacuum" because it is an impregnation process for preparing the micro-catalyst of the present invention which avoids the use of any solvent during the catalyst preparation.

In a preferred embodiment, the nickel precursor salt is nickel (II) nitrate hexahydrate.

In another preferred embodiment, the metal salt precursor of cerium oxide is cerium (III) nitrate hexahydrate and the metal salt precursor of lanthanum oxide is lanthanum (III) nitrate hexahydrate.

In a further preferred embodiment, the mixture in step b) is preferably carried out with low stirring, preferably at 50-150 r.p.m, and at room temperature for 10-15 min.

In a further preferred embodiment, step c) is preferably carried out under low stirring, more preferably at a temperature of 110-130 °C under low stirring, preferably at 50-150 r.p.m.

In a further preferred embodiment, step f) is carried out with a temperature ramp of 1 °C·min ⁻¹ and a hold time of 30 min.

In a further preferred embodiment, the hydrogen is present in the stream in step g) at 5-100 vol. %, being the rest, if present, preferably argon or nitrogen.

In a third aspect, the present invention relates to the use of the micro-catalyst according to first aspect of the invention including all the embodiments alone or in combination, for producing synthetic methane by the carbon dioxide and hydrogen conversion into methane and water.

In a preferred embodiment, the carbon dioxide is pure (i.e. >99 vol.%) or is present in a gas mixture, for example, a gas mixture as downstream of processes such as (anaerobic digestion, combustion, incineration or gasification). Said gas mixture could also be a biogas. A biogas is usually defined as a mixture of gases produced by the decomposition of organic matter in the absence of oxygen, comprising methane and carbon dioxide and other gases and contaminants, for example H₂S. Before using, contaminants and other gases are usually removed so that the biogas consists essentially of methane and carbon dioxide.

### EXAMPLES

The present invention is further disclosed by way of examples which only intend to illustrate the present invention and by no means are limiting the scope thereof.

### Example 1

This example shows the advantages of the process of the present invention.

300 mg of micro-catalyst was filled in an electrically heated fixed-bed reactor, diluted with 3 g of silicon carbide of similar particle size (355 µm) under isothermal conditions. The reaction temperature was monitored using a K-type thermocouple placed in the middle of the catalyst bed. The reactants were flowed by mass flow controllers at 200 N·mL·min⁻¹ with stoichiometric H₂:CO₂ molar ratio of 4. Gas Hourly Space Velocity, calculated as the ratio of inlet flow rate in standard conditions to the volume of catalyst, was set at 50.000 h⁻¹. Pressure was set at the reactor outlet by an automatic valve at 5 bar·g. After reaction, the products passed through a cold liquid-gas separator (5 °C), where water was trapped, and then the dry flow was measured by a mass flow meter. The composition of the dry gas was analysed by an on-line gas micro-chromatograph.

Typical conversion of the products at the outlet is 25% at 250 °C, 46% at 275 °C, 85% at 300 °C, 95% at 325 °C and 98% at 350 °C. Typical composition of the products at the outlet is 2.7% CO₂, 11% H₂ and 10% CH₄ at 300 °C; and 1% CO₂, 4% H₂ and 20% CH₄ at 350 °C. Figure 1 shows the advantage of the preparation method. MIV method showed higher conversion values, compared to its analogous conventional method MI, at the whole range of temperatures. When compared to conventional WET, in addition to the benefits of using a non-solvent method, MIV showed similar conversion values and even higher at the range of 285-330 °C.

### Example 2

This example shows several formulations of the micro-catalyst of the present invention. The compositions including ruthenium are not in the scope of the present invention and are provided for comparative purposes.

A series of micro-catalysts were prepared by vacuum infiltration method using the following composition, being the rest γ-Al₂O₃ mesoporous support.
A. Ni (20 wt.%) and La₂O₃ (15 wt.%)
B. Ni (20 wt.%) and CeO₂ (15 wt.%)
C. Ni (20 wt.%), CeO₂ (7.5 wt.%) and La₂O₃ (7.5 wt.%)
D. Ni (20 wt.%), CeO₂ (15 wt.%) and Ru (5 wt.%)
E. Ni (10 wt.%), CeO₂ (7.5 wt.%), La₂O₃ (7.5 wt.%) and Ru (10 wt.%)

The series of catalyst were characterised as follows.
SEM imaging, mapping and elemental composition analysis were conducted at 20 kV using a scanning electron microscope (SU-70 Hitachi) equipped with an energy dispersive X-rays spectroscopy detector (EDX, Oxford Instruments). The as-received catalyst samples from the reactors were gently crushed prior to SEM-EDX analysis to better observe the inner parts of the used catalyst. No coating was necessary since the samples are of conductive nature. The chemical composition analysis was restricted to Ni, Ru, Al, Ce, La and O, and it was computed as the average over ten measurements on different regions for each powder sample. A copper standard was used for the system calibration.
The composition is detailed in Table 1.

**Table 1. Catalyst composition analysed by EDX (wt.%)**

| catalyst | Ni | Ce | La | Ru | Al | O |
|---|---|---|---|---|---|---|
| A | 22.65 | 15.17 | | | 24.18 | 38.00 |
| B | 22.05 | | 6.75 | | 28.54 | 42.58 |
| C | 22.16 | 8.16 | 3.85 | | 25.21 | 40.62 |
| D | 20.02 | 11.81 | | 3.12 | 24.70 | 37.40 |
| E | 11.58 | 6.32 | 3.87 | 4.68 | 26.50 | 39.75 |

Figure 2 illustrates an image of the micro-catalyst developed, which shows an average particle diameter of the micro-spheres of 266 µm. Figure 3 shows an example of the method used for the determination of the elemental composition, whose results are listed in Table 1, indicating the presence of Ni, Ce, Al and O elements.

The true or skeletal density of the series of catalysts was measured in an Ultrapyc pycnometer 1200e, (Quantachrome Instruments) with helium as the probe gas. The helium pycnometer is equipped with three different size sample cell (small, medium and large). In order to prevent fine materials from being displaced by helium gas during measurements, the large sample cell with its fitted cap was used. Approximately the 75% of the sample cell volume was filled by each sample to ensure accuracy. The loaded sample cell was then transferred to the sample chamber and each measurement was performed by dosing the sample chamber with helium (to ≈ 20 psi), which was after allowed to expand into the reference chamber. True density as shown was determined by the average of collected data points from three runs. Table 2 shows the true density of the series of catalysts. True density of the catalysts is in the range of 3.51-3.84 g/cm³.

**Table 2. Catalyst true density**

| catalyst | ρ (g/cm³) |
|---|---|
| A | 3.84 |
| B | 3.69 |
| C | 3.75 |
| D | 3.61 |
| E | 3.51 |

N₂-physisorption (adsorption/desorption) measurements were determined at liquid nitrogen temperature using an automated TriStar II 3020-Micromeritics analyser. Samples were degassed at 90 °C for 1 h, and then at 250 °C for 4 h in a FlowPrep 060-Micromeritics. Brunauer-Emmett-Teller (BET) method was used to calculate the BET surface area for a relative pressure (P·Pₒ⁻¹) range of 0.05-0.30. Barrett-Joyner-Halenda (BJH) method was applied to desorption branch of the isotherms to determine the average pore size and the total pore volume, which was calculated from the maximum adsorption value at P·Pₒ⁻¹ = 0.999. Figure 4 shows the N₂-physisorption isotherms and Figure 5 the pore diameter distribution of samples A, B, C, D, E. Porosity values obtained by this technique are listed in Table 3.

**Table 3. Porosity of the catalysts: surface area, pore volume and pore diameter.**

| catalyst | S_{BET} [m²/g] | Vₚ [cm³/g] | dₚ [nm] |
|---|---|---|---|
| A | 148.24 | 0.26 | 6.31 |
| B | 148.89 | 0.25 | 6.13 |
| C | 136.49 | 0.24 | 5.98 |
| D | 161.82 | 0.24 | 5.82 |
| E | 167.49 | 0.25 | 5.59 |

Reducibility of the catalysts was analysed by temperature programmed reduction method (H₂-TPR) on an Authochem (Micromeritics). In each run, 70 mg of fresh calcined catalyst was used for the analysis. The H₂-TPR measurements were conducted using 12 vol.% H₂/Ar at flow of 50 NmL·min⁻¹ in the temperature range of 35 to 800 °C at a heating ramp of 10 °C·min⁻¹. The H₂ uptake was measured with a thermal conductivity detector (TCD). Before the detector, the gas flow was passed through a cold trap (composed of ice NaCl) to condense the water generated during the experiment. A standard curve was used to calibrate the degree reduction of Ni species. The amount of reduced NiO to Ni⁰ was calculated by integrating the reduction peaks in the H₂-TPR profiles and expressed as a percentage of consumption to reduce the Ni species in the catalysts. Reducibility results are shown in Figure 6.

XRD patterns were collected within the 2θ range 20-80° in a Bruker type XRD D8 Advance A25 diffractometer using a Cu Kα radiation (λ = 1.5406 Å), a voltage of 40 kV, a current of 40 mA and a step size of 0.05° (with 3 s duration at each step). The average crystal sizes of the metallic nickel (NiO), ruthenium (RuO₂) and cerium oxide (CeO₂) were estimated using the Scherrer's equation at the most intense peaks; 2θ=37.2 for Ni (111) 2θ=35.2 for RuO₂ (101) and 28.60° for CeO₂ (220): D=(Kλ!βCosθ), where λ is the X-ray wavelength, β is the full width of the diffraction line at half maximum (FWHM), and θ is the Bragg angle. Reducibility results are shown in Figure 7.

CO-Chemisorption was performed on a chemisorption analyser (Autochem HP-Micromeritics). Before measurements, samples (ca. 50 mg) were reduced using 50 NmL·min⁻¹ in a 12 vol.% H₂/Ar flow at 500 °C for 3 h and a heating ramp of 1 °C·min⁻¹. Then, CO-Chemisorption was measured at 35 °C under a 10 vol.% CO/He flow. CO pulses were periodically introduced until saturation was reached. Nickel metal surface area and dispersion were calculated assuming the stoichiometric factor for CO to Ni equal to unity, atomic weight of 58.71, atomic cross-sectional area of 0.0649 nm² and density of 8.90 g.cm⁻³. The fresh sample was measured after calcination and after a reduction in a tubular furnace using 100 NmL·min⁻¹ in a 5 vol.% H₂/Ar flow at 500 °C for 3 h with a heating and cooling ramp of 1 °C·min⁻¹. Results are listed in Table 4.

**Table 4. Metallic surface area**

| catalyst | Active Metal Surface Area [m²/g cat.] |
|---|---|
| A | 1.64 |
| B | 1.72 |
| C | 2.08 |
| D | 2.21 |
| E | 1.81 |

### Example 3

This example shows how the compositions according to the present invention are advantageously useful. The compositions including ruthenium are not in the scope of the present invention and are provided for comparative purposes.

A series of micro-catalyst were prepared by vacuum infiltration method using the following composition, being the rest γ-Al₂O₃ mesoporous support.
A. Ni (20 wt.%) and La₂O₃ (15 wt.%)
B. Ni (20 wt.%) and CeO₂ (15 wt.%)
C. Ni (20 wt.%), CeO₂ (7.5 wt.%) and La₂O₃ (7.5 wt.%)
D. Ni (20 wt.%), CeO₂ (15 wt.%) and Ru (5 wt.%)
E. Ni (10 wt.%), CeO₂ (7.5 wt.%), La₂O₃ (7.5 wt.%) and Ru (10 wt.%)

The catalytic activity on CO₂ methanation was evaluated as Example 1. In Figure 8, it is compared the catalytic activity of the different sample composition. As observed, sample A, B and C exhibited higher conversion values than sample D and E.

### Example 4

This example shows the advantages of using a micro-catalyst vs. macrocatalyst.

The catalytic activity of the micro-catalyst (100-300 µm) and a macro-catalyst (2-3 mm) were prepared using the melting infiltration method. The catalytic activity was measured as Example 1.

Figure 9 compared the catalytic activity of a micro-catalyst versus a macro-catalyst. It is observed that the activity outstands the activity of the macro-catalyst. The particle size distribution of the micro-catalyst is shown in Figure 10.

## Claims

1. A micro-catalyst consisting of nickel (Ni) and a compound selected from cerium oxide (CeO₂), lanthanum oxide (La₂O₃) or a combination thereof, using aluminium oxide (γ-Al₂O₃) as a support, having said micro-catalyst an average particle size between 100 and 900 µm, wherein the Ni and said compound selected from CeO₂, La₂O₃ or a combination thereof have a metal precursor salt with a melting point below 150°C and lower than its decomposition temperature, being the metal Ni or Ce o La.

2. The micro-catalyst according to claim 1, wherein the Ni is present at 10-30 wt % and the combination of CeO₂ and La₂O₃ is present with a total amount corresponding to 5-25 wt % with respect to the total composition, being the rest aluminium oxide (γ-Al₂O₃).

3. The micro-catalyst according to claim 2, wherein the Ni is present at 15-25 wt % and the combination of CeO₂ and La₂O₃ is present with a total amount corresponding to 15-20 wt % with respect to the total composition, being the rest aluminium oxide (γ-Al₂O₃).

4. The micro-catalyst according to any of claims 1 to 3, having a surface area of 100-200 m²/g.

5. The micro-catalyst according to any of claims 1 to 4, having a pore volume of 0.2-0.4 cm³/g.

6. The micro-catalyst according to any of claims 1 to 4, having a pore size of 2-50 nm, preferably of 5-9 nm.

7. A process for preparing a micro-catalyst according to any of claims 1 to 6, comprising the following steps:
a) preparing the micro-catalyst support by drying γ-Al₂O₃ at 90-110 °C;
b) mixing the metal precursors salt as defined in claim 1 of nickel and a compound selected from cerium oxide (CeO₂), lanthanum oxide (La₂O₃) or a combination thereof, with the support of step a);
c) heating the mixture of step b) at a temperature of 80-150 °C;
d) cooling down the mixture of step c) at 70-90 °C;
e) drying the mixture of step d) at 70-90 °C under vacuum for 3-15 hours.
f) heating the mixture obtained in step e) at 400-500 °C under air;
g) activating the mixture as obtained in step f) by flowing a stream comprising hydrogen at 300-500°C.

8. The micro-catalyst according to any of claims 1 to 6 or the process according to claim 7, wherein the anion of the metal precursor salt is selected from nitrate hydrate, chloride, acetate, sulphate, ammonium sulfate, iodate, oxalate and perchlorate.

9. The micro-catalyst according to any of claims 1 to 6 or the process according to claim 7 or 8, wherein the nickel precursor is nickel (II) nitrate hexahydrate.

10. The micro-catalyst according to any of claims 1 to 6 or the process according to any of claims 7 to 9, wherein the metal salt precursor of cerium oxide is cerium (III) nitrate hexahydrate and the metal salt precursor of lanthanum oxide is lanthanum (III) nitrate hexahydrate.

11. The process according to any of claims 7 to 10, wherein the mixture in step b) is carried out at 50-150 r.p.m. and at room temperature for 10-15 min.

12. The process according to any of claims 7 to 11, wherein step f) is carried out with a temperature ramp of 1°C/min⁻¹ and a hold time of 30 min.

13. The process according to any of claims 7 to 12, wherein the hydrogen is present in the stream in step g) at 5-100 vol. %.

14. Use of the micro-catalyst according to any of claims 1 to 6 or 8 to 10, for producing synthetic methane by the carbon dioxide and hydrogen conversion into methane and water.

15. The use according to claim 14, wherein the carbon dioxide is pure or is present in a gas mixture.
